# EUROPEAN PATENT APPLICATION

(11) **EP 1 978 104 A1**
(43) Date of publication of application: **08.10.2008**
(21) Application number: 07006897.8
(22) Date of filing: 03.04.2007
(51) Int. Cl.: C12Q 1/68

(54) **A method for the quantitative analysis of RNA molecules in a sample**

(71) Applicant: Freie Universität Berlin, 14195 Berlin (DE)
(72) Inventor: Sharbati-Tehrani, Soroush, Dr., 10587 Berlin (DE); Einspanier, Ralf, Prof. Dr., 14532 Kleinmachnow (DE)
(74) Representative: Czychowski, Christian A.

(57) **Abstract**

The invention relates to a method for the quantitative analysis of RNA molecules comprising the following steps:
a) performing a reverse transcription reaction with a first oligonucleotide molecule to yield cDNA molecules, wherein the first oligonucleotide molecule comprises
- a first sequence portion that hybridizes with the RNA molecules at,
- a second sequence portion that provides for a sequence for binding of a third oligonucleotide to prime an enzymatic DNA amplification reaction,

b) performing an enzymatic DNA amplification reaction to amplify the cDNA molecules, comprising:
- a second oligonucleotide molecule, comprising a first sequence portion for hybridizing with the cDNA molecules, and a second sequence portion that provides for a sequence for binding of a fourth oligonucleotide to prime the enzymatic DNA amplification reaction,
- a third oligonucleotide molecule for priming an enzymatic DNA amplification reaction, and
- a fourth oligonucleotide molecule for priming an enzymatic DNA amplification reaction, and

c) performing a quantitative analysis of the amplified DNA molecules.

SEQUENCE LISTING

<110>  Freie Universität Berlin

<120>  A method for the quantitative analysis of RNA molecules in a
       sample

<130>  FBP18555

<160>  50

<170>  PatentIn version 3.3

<210>  1
<211>  24
<212>  RNA
<213>  Homo sapiens

<400>  1
guccaguuuu cccaggaauc ccuu                                              24


<210>  2
<211>  36
<212>  DNA
<213>  Artificial

<220>
<223>  Oligonucleotide

<400>  2
tgtcaggcaa ccgtattcac cgtgagtggt aaggga                                 36


<210>  3
<211>  55
<212>  DNA
<213>  Artificial

<220>
<223>  Oligonucleotide

<400>  3
cgtcagatgt ccgagtagag ggggaacggc ggtccagttt tcccaggaat ccctt            55


<210>  4
<211>  49
<212>  DNA
<213>  Artificial

<220>
<223>  Oligonucleotide

<400>  4
cgtcagatgt ccgagtagag ggggaacggc ggtccagttt tcccaggaa                   49


<210>  5
<211>  21
<212>  DNA
<213>  Artificial

<220>
<223>  Oligonucleotide

<400>  5
tgtcaggcaa ccgtattcac c                                                 21

<210> 6
<211> 21
<212> DNA
<213> Artificial

<220>
<223> Oligonucleotide

<400> 6
cgtcagatgt ccgagtagag g 21


<210> 7
<211> 22
<212> RNA
<213> Homo sapiens

<400> 7
ugagguagua gguuguauag uu 22


<210> 8
<211> 36
<212> DNA
<213> Artificial

<220>
<223> Oligonucleotide

<400> 8
tgtcaggcaa ccgtattcac cgtgagtggt aactat 36


<210> 9
<211> 53
<212> DNA
<213> Artificial

<220>
<223> Oligonucleotide

<400> 9
cgtcagatgt ccgagtagag ggggaacggc gtgaggtagt aggttgtata gtt 53


<210> 10
<211> 50
<212> DNA
<213> Artificial

<220>
<223> Oligonucleotide

<400> 10
cgtcagatgt ccgagtagag ggggaacggc gtgaggtagt aggttgtata 50


<210> 11
<211> 22
<212> RNA
<213> Homo sapiens

<400> 11
uagcuuauca gacugauguu ga 22


<210> 12
<211> 36

<212>  DNA
<213>  Artificial

<220>
<223>  Oligonucleotide

<400>  12
tgtcaggcaa ccgtattcac cgtgagtggt tcaaca                                36


<210>  13
<211>  53
<212>  DNA
<213>  Artificial

<220>
<223>  Oligonucleotide

<400>  13
cgtcagatgt ccgagtagag ggggaacggc gtagcttatc agactgatgt tga              53


<210>  14
<211>  47
<212>  DNA
<213>  Artificial

<220>
<223>  Oligonucleotide

<400>  14
cgtcagatgt ccgagtagag ggggaacggc gtagcttatc agactga                     47


<210>  15
<211>  22
<212>  RNA
<213>  Homo sapiens

<400>  15
ugagguagua gguugugugg uu                                               22


<210>  16
<211>  36
<212>  DNA
<213>  Artificial

<220>
<223>  Oligonucleotide

<400>  16
tgtcaggcaa ccgtattcac cgtgagtggt aaccac                                36


<210>  17
<211>  53
<212>  DNA
<213>  Artificial

<220>
<223>  Oligonucleotide

<400>  17
cgtcagatgt ccgagtagag ggggaacggc gtgaggtagt aggttgtgtg gtt              53


<210>  18
<211>  50

<212>  DNA
<213>  Artificial

<220>
<223>  Oligonucleotide

<400>  18
cgtcagatgt ccgagtagag ggggaacggc gtgaggtagt aggttgtgtg                50


<210>  19
<211>  22
<212>  RNA
<213>  Homo sapiens

<400>  19
ugagguagua gguuguaugg uu                                              22


<210>  20
<211>  36
<212>  DNA
<213>  Artificial

<220>
<223>  Oligonucleotide

<400>  20
tgtcaggcaa ccgtattcac cgtgagtggt aaccat                               36


<210>  21
<211>  53
<212>  DNA
<213>  Artificial

<220>
<223>  Oligonucleotide

<400>  21
cgtcagatgt ccgagtagag ggggaacggc gtgaggtagt aggttgtatg gtt            53


<210>  22
<211>  50
<212>  DNA
<213>  Artificial

<220>
<223>  Oligonucleotide

<400>  22
cgtcagatgt ccgagtagag ggggaacggc gtgaggtagt aggttgtatg                50


<210>  23
<211>  22
<212>  RNA
<213>  Homo sapiens

<400>  23
uagcagcacg uaaauauugg cg                                              22


<210>  24
<211>  36
<212>  DNA
<213>  Artificial

<220>
<223>  Oligonucleotide

<400>  24
tgtcaggcaa ccgtattcac cgtgagtggt cgccaa                                36

<210>  25
<211>  53
<212>  DNA
<213>  Artificial

<220>
<223>  Oligonucleotide

<400>  25
cgtcagatgt ccgagtagag ggggaacggc gtagcagcac gtaaatattg gcg              53

<210>  26
<211>  47
<212>  DNA
<213>  Artificial

<220>
<223>  Oligonucleotide

<400>  26
cgtcagatgt ccgagtagag ggggaacggc gtagcagcac gtaaata                     47

<210>  27
<211>  21
<212>  RNA
<213>  Homo sapiens

<400>  27
aucacauugc cagggauuac c                                                21

<210>  28
<211>  36
<212>  DNA
<213>  Artificial

<220>
<223>  Oligonucleotide

<400>  28
tgtcaggcaa ccgtattcac cgtgagtgct ggtaat                                36

<210>  29
<211>  54
<212>  DNA
<213>  Artificial

<220>
<223>  Oligonucleotide

<400>  29
cgtcagatgt ccgagtagag ggggaacggc gatcacattg ccagggatta ccag             54

<210>  30
<211>  46
<212>  DNA
<213>  Artificial

<220>
<223>  Oligonucleotide

<400>  30
cgtcagatgt ccgagtagag ggggaacggc gatcacattg ccaggg                    46


<210>  31
<211>  21
<212>  RNA
<213>  Homo sapiens

<400>  31
uucacagugg cuaaguuccg c                                               21


<210>  32
<211>  36
<212>  DNA
<213>  Artificial

<220>
<223>  Oligonucleotide

<400>  32
tgtcaggcaa ccgtattcac cgtgagtggt gcggaa                               36


<210>  33
<211>  52
<212>  DNA
<213>  Artificial

<220>
<223>  Oligonucleotide

<400>  33
cgtcagatgt ccgagtagag ggggaacggc gttcacagtg gctaagttcc gc             52


<210>  34
<211>  46
<212>  DNA
<213>  Artificial

<220>
<223>  Oligonucleotide

<400>  34
cgtcagatgt ccgagtagag ggggaacggc gttcacagtg gctaag                    46


<210>  35
<211>  22
<212>  RNA
<213>  Homo sapiens

<400>  35
uaacacuguc ugguaaagau gg                                              22


<210>  36
<211>  36
<212>  DNA
<213>  Artificial

<220>
<223>  Oligonucleotide

<400>  36
tgtcaggcaa ccgtattcac cgtgagtggt ccatct                                36

<210>  37
<211>  53
<212>  DNA
<213>  Artificial

<220>
<223>  Oligonucleotide

<400>  37
cgtcagatgt ccgagtagag ggggaacggc gtaacactgt ctggtaaaga tgg             53

<210>  38
<211>  47
<212>  DNA
<213>  Artificial

<220>
<223>  Oligonucleotide

<400>  38
cgtcagatgt ccgagtagag ggggaacggc gtaacactgt ctggtaa                    47

<210>  39
<211>  22
<212>  RNA
<213>  Homo sapiens

<400>  39
ugagaugaag cacuguagcu ca                                               22

<210>  40
<211>  36
<212>  DNA
<213>  Artificial

<220>
<223>  Oligonucleotide

<400>  40
tgtcaggcaa ccgtattcac cgtgagtggt tgagct                                36

<210>  41
<211>  53
<212>  DNA
<213>  Artificial

<220>
<223>  Oligonucleotide

<400>  41
cgtcagatgt ccgagtagag ggggaacggc gtgagatgaa gcactgtagc tca             53

<210>  42
<211>  47
<212>  DNA
<213>  Artificial

<220>
<223>  Oligonucleotide

<400> 42
cgtcagatgt ccgagtagag ggggaacggc gtgagatgaa gcactgt 47

<210> 43
<211> 22
<212> RNA
<213> Homo sapiens

<400> 43
caacggaauc ccaaaagcag cu 22

<210> 44
<211> 36
<212> DNA
<213> Artificial

<220>
<223> Oligonucleotide

<400> 44
tgtcaggcaa ccgtattcac cgtgagtggt agctgc 36

<210> 45
<211> 53
<212> DNA
<213> Artificial

<220>
<223> Oligonucleotide

<400> 45
cgtcagatgt ccgagtagag ggggaacggc gcaacggaat cccaaaagca gct 53

<210> 46
<211> 47
<212> DNA
<213> Artificial

<220>
<223> Oligonucleotide

<400> 46
cgtcagatgt ccgagtagag ggggaacggc gcaacggaat cccaaaa 47

<210> 47
<211> 22
<212> RNA
<213> Homo sapiens

<400> 47
uaauacugcc ggguaaugau gg 22

<210> 48
<211> 36
<212> DNA
<213> Artificial

<220>
<223> Oligonucleotide

<400> 48
tgtcaggcaa ccgtattcac cgtgagtggt ccatca 36

<210>  49
<211>  53
<212>  DNA
<213>  Artificial

<220>
<223>  Oligonucleotide

<400>  49
cgtcagatgt ccgagtagag ggggaacggc gtaatactgc cgggtaatga tgg          53

<210>  50
<211>  47
<212>  DNA
<213>  Artificial

<220>
<223>  Oligonucleotide

<400>  50
cgtcagatgt ccgagtagag ggggaacggc gtaatactgc cgggtaa          47

## Description

The invention relates to a method and to a kit for the quantitative analysis of RNA molecules, particularly small RNA molecules in a biological sample. The invention further relates to the use of such a method for the diagnosis and/or prognosis of at least one disease, and to oligonucleotide molecules for the quantitative analysis of RNA molecules.

### Background of the invention

RNA interference (RNAi) is an evolutionarily conserved process that functions to inhibit gene expression (Bemstein et al. (2001), Nature 409: 363-6; Dykvhoom et al. (2003) Nat. Rev. Mol. Cell. Biol. 4: 457-67). The phenomenon of RNAi was first described in *Caenorhabditis elegans,* where injection of double-stranded RNA (dsRNA) leads to efficient sequence-specific gene silencing of the mRNA that was complementary to the dsRNA (Fire et al. (1998) Nature 391: 806-811). RNAi has also been described in plants as a phenomenon called post-transcriptional gene silencing (PTGS), which is likely used as a viral defense mechanism (Jorgensen (1990) Trends Biotechnol. 8: 340-4; Brigneti et al. (1998) EMBO J. 17: 6739-46; Hamilton and Baulcombe (1999) Science 286: 950-952).

An early indication that the molecules that regulate PTGS were short RNAs processed from longer dsRNA was the identification of short 21 to 22 nucleotide dsRNA derived from the longer dsRNA in plants (Hamilton and Baulcombe (1999) Science 286: 950-952). Based on these observations, it was tested if synthetic 21 to 25 nucleotide long synthetic dsRNA molecules function to specifically inhibit gene expression in Drosophila embryo lysates and mammalian cell culture (Elbashir et al. (2001) Nature 411: 494-498; Elbashir et al. (2001) EMBO J. 20: 6877-6888; Elbashir et al. (2001) Genes Dev. 15: 188-200). It was demonstrated that small interfering RNAs (siRNAs) had the ability to specifically inhibit gene expression in mammalian cell culture without induction of the interferon response.

MicroRNA molecules (miRNAs) regulate gene expression by catalyzing the cleavage of messenger RNA (mRNA) (Hutvagner and Zamore (2002) Science, 297, 2056-2060; Zhang et al. (2002) EMBO J., 21, 5875-5885; Zhang, H et al. (2004) Cell, 118, 57-68; Zhang and Semino(2003) Adv. Exp. Med. Biol., 534, 147-163; Doench et al. (2003) Genes Dev., 17, 438-442; Doench and Sharp (2004) Genes Dev., 18, 504-511) or repressing mRNA translation (Doench and Sharp (2004) Genes Dev., 18, 504-511; Wightman et al. (1993) Cell, 75, 855-862; Olsen and Ambros (1999) Dev. Biol., 216, 671-680).

Short RNA molecules are difficult to quantitate. Although miRNAs represent a relatively abundant class of transcripts, their expression levels vary greatly among species and tissues (Kimet al. (2004) Proc. Natl Acad. Sci. USA, 101, 360-365). Less abundant miRNAs routinely escape detection with technologies such as cloning, northern hybridization (Lim et al. (2003) Science, 299, 1540) and microarray analysis (Krichevsky et al. (2003) [Erratum (2004) RNA, 10, 551.]. RNA, 9, 1274-1281.; Liu et al. (2004) Proc. Natl Acad. Sci. USA, 101, 9740-9744). For example, with respect to the use of the polymerase chain reaction (PCR) to amplify and measure the small RNA molecules, most PCR primers are longer than the small RNA molecules, and so it is difficult to design a primer that has significant overlap with a small RNA molecule, and that selectively hybridizes to the small RNA molecule.

Therefore, the problem underlying the present invention was to provide a means for the quantitative detection of small RNA molecules in a sample.

### Description of the invention

The problem is solved by providing a method for the quantitative analysis of RNA molecules in a sample according to the present invention. Such a method comprises the following steps:

First, a reverse transcription reaction is performed using a first oligonucleotide molecule. This reverse transcription reaction can also be referred to as a primer extension reaction.

Through this reaction, DNA molecules are generated that are reverse complementary in sequence to the template RNA molecules, and which are referred to as cDNA molecules.

Said first oligonucleotide molecule comprises at least two sequence portions, namely a first sequence portion that is complementary to a sequence portion of the RNA molecule, preferably to the 3' end of the RNA molecule, and therefore hybridizes with the template RNA molecules to be analyzed. This first sequence portion is preferably located at the 3' end of the first oligonucleotide molecule. Said first oligonucleotide molecule also comprises a second sequence portion that provides for a sequence for binding of a third oligonucleotide to prime an enzymatic DNA amplification reaction. This second sequence portion is preferably located at the 5' end of the first oligonucleotide molecule.

Secondly, an enzymatic DNA amplification reaction is performed to amplify the cDNA molecules. This amplification is performed in the presence of the following oligonucleotides.

A second oligonucleotide molecule is used, which comprises a first sequence portion, preferably at its 3' end, that is corresponding in sequence to the RNA molecule. Therefore, the second oligonucleotide can hybridize with the cDNA molecules formed in the first step. In addition, a second sequence portion of the second oligonucleotide provides for a sequence for binding of a fourth oligonucleotide, which can be used to prime an enzymatic DNA amplification reaction. This second sequence portion is preferably located at the 5' end of the second oligonucleotide.

The enzymatic DNA amplification reaction is further performed in the presence of a third and a fourth oligonucleotide molecule for priming an enzymatic DNA amplification reaction. The third oligonucleotide molecule hybridizes with a sequence that is reverse complementary to the second sequence portion of the first oligonucleotide molecule, which is provided in the DNA amplification reaction primed by the second oligonucleotide molecule. The fourth oligonucleotide molecule hybridizes with a sequence that is reverse complementary to the second sequence portion of the second oligonucleotide molecule, which is provided in the DNA amplification reaction primed by the resulting cDNA molecule after the reverse transcription. When hybridized to the template, the third and the fourth oligonucleotide molecule are oriented in opposite orientation on different strands of the template DNA, allowing for the amplification of the template DNA.

Thirdly, a quantitative analysis of the amplified DNA molecules is performed to deduce the number or concentration of RNA molecules in the sample.

In essence, the present invention provides a method for the quantitative analysis of RNA molecules in a sample in which a first step of a reverse transcription of the RNA using a first oligonucleotide (primer) is combined with a following amplification step using three oligonucleotides (primers), namely one sequence specific primer (second oligonucleotide molecule) and two primers (third and fourth oligonucleotide molecule) which hybridize with a sequence that was introduced into the DNA using the sequence specific first and second primers.

Surprisingly, this method was shown to yield linear results over six to eight logarithmic steps.

In a preferred embodiment of the invention, the second sequence portion of the first and/or second oligonucleotide molecule is a universal primer binding site. To this universal primer binding site, a universal primer can bind. The third and/or fourth oligonucleotide molecule can be designed as such universal primers. Here, the term "universal primer" is meant to comprise a primer with a sequence that can bind to more than one kind of first and second oligonucleotide molecules, so that it is possible to analyze more than one RNA species in one experiment. In order to perform multiplex experiments, analyzing more than one different RNA molecule simultaneously, a set of first oligonucleotide molecules is used in which each species of first sequence portions is sequence specific for a particular RNA species. This makes a good quantitative comparison of the abundance of different RNA species in a sample possible.

It is possible that the first and second sequence portion of the first oligonucleotide molecule and/or the first and second sequence portion of the second oligonucleotide molecule are separate from each other, i.e. not overlapping. It may, however, be also possible to have the first and second sequence portion overlap (see, e.g. primers used in example III). The oligonucleotides can be constructed such that spacer regions are located between the respective first and second sequence portions. Such spacer regions can be between one and 30 nucleotides (nt) in length.

The first sequence portion of the first oligonucleotide is preferably between 4 and 10 nt long. The length of the first sequence portion of the second oligonucleotide is dependent on the length of the RNA template. The universal primers are preferably between 15 and 30 nt, more preferably between 18 and 25 nt, and most preferably between 19 and 22 nt long.

When analyzing very short RNA molecules, which are shorter in length than the oligonucleotides themselves, it is preferred that at least the second sequence portion of the first oligonucleotide molecule forms a 5' overhang when the first oligonucleotide molecule is hybridized with the RNA molecule. It is similarly preferred that the second sequence portion of the second oligonucleotide molecule forms a 5' overhang when the second oligonucleotide molecule is hybridized with the cDNA molecule generated in the reverse transcription (primer extension) step.

To quantitate the number of RNA molecules in the sample, it is preferred in one embodiment of the present invention that the enzymatic DNA amplification reaction is performed in the presence of a fluorescent dye, which binds to double-stranded DNA (e.g. SYBR green) and generates a real-time signal. The enzymatic DNA amplification reaction can also be performed in the presence of at least one kind of probe that generates a real-time signal, e.g. a TaqMan^{®} probe, a molecular beacon or other probes, which are known to a person of skill in the art. The detection of the signal stemming from such a probe is used to quantitate the amount of RNA molecules in the sample using a real-time amplification assay. Using different kinds of probes simultaneously is advantageous in a multiplex approach.

It is preferred that the enzymatic DNA amplification reaction is a polymerase chain reaction (PCR).

It is of particular advantage to use the present invention for the analysis of microRNA (miRNA), preferably from mammals, in particular from humans. Since these molecules are about 20 nt to 22 nt in length, it is preferred to use a first and second oligonucleotide molecule with a 5' overhang, as described above. It is furthermore preferred that the quantitative analysis is performed by generating a real-time signal using the DNA-binding fluorescent dye SYBR green. The signals from the amplified DNA molecules are measured and compared to a standard, preferably an internal RNA-standard.

The present invention also pertains to the use of the method as described above for the diagnosis and/or prognosis of at least one disease and/or disorder, such as cancer. Such diseases and disorders related to pathological miRNA concentrations in the cell are known to a person of skill in the art.

Recent studies indicate a basic role of miRNAs in immunity in particular in response to components of bacterial pathogens (Taganov et al. (2007) Immunity, 26, 133-137). In many recent studies, dysregulation of miRNA expression is related to cancer. Michael et al. ((2007) Mol Cancer Res, 1, 882-891), e.g., showed a decreased expression of miR-143 and miR-145 in colorectal neoplasia. In another study it was shown that expression of the oncogene RAS is regulated by the let-7 family (Johnson et al. (2005) Cell, 120, 635-647).

The diagnosis is based on the comparison of concentrations of miRNA in a sample from a patient to concentrations of miRNA in a sample from one or more healthy individuals.

The problem as stated earlier is also solved by providing a kit for the quantitative detection of RNA molecules, particularly small RNA molecules in a sample, comprising a first oligonucleotide molecule, which comprises a first sequence portion that is complementary to a sequence portion, preferably the 3' end of the RNA molecule and therefore hybridizes with the RNA molecules, and a second sequence portion that provides for a sequence for binding of a third oligonucleotide to prime an enzymatic DNA amplification reaction.

The kit further comprises a first enzyme catalyzing the reverse transcription of the RNA molecules into cDNA molecules (also referred to as primer extension reaction), which is preferably a reverse transcriptase from *moloney murine leukemia virus* (M-MuLV) with or without ribonuclease H activity, and a second enzyme catalyzing an amplification reaction, preferably a DNA polymerase from *Thermus aquaticus* for performing a polymerase chain reaction (PCR).

The kit further comprises a second oligonucleotide molecule, comprising a first sequence portion that is complementary to a sequence portion of the RNA molecule, preferably to the 3' end for hybridizing with the cDNA molecules, and a second sequence portion that provides a sequence for binding of a fourth oligonucleotide, as well as a third and fourth oligonucleotide molecule for priming an enzymatic DNA amplification reaction.

It is preferred that at least the second sequence portion of the first oligonucleotide molecule forms a 5' overhang when the first oligonucleotide molecule is hybridized with an RNA molecule, and that the second sequence portion of the second oligonucleotide molecule forms a 5' overhang when the second oligonucleotide molecule is hybridized with the cDNA molecule formed in a reverse transcription reaction using the first oligonucleotide. Furthermore, the underlying problem mentioned above is also solved by providing an oligonucleotide for the quantitative analysis of RNA molecules, particularly small RNA molecules in a sample, selected from the group consisting of SEQ ID NOs 2 to 6, 8 to 10, 12 to 14, 16 to 18, 20 to 22, 24 to 26, 28 to 30, 32 to 34, 36 to 38, 40 to 42, 44 to 46, and 48 to 50. These oligonucleotides can be used for the quantitative analysis of RNA molecules in particular small RNA molecules, such as miRNA molecules.

The invention is now described with reference to the drawings of the figures.

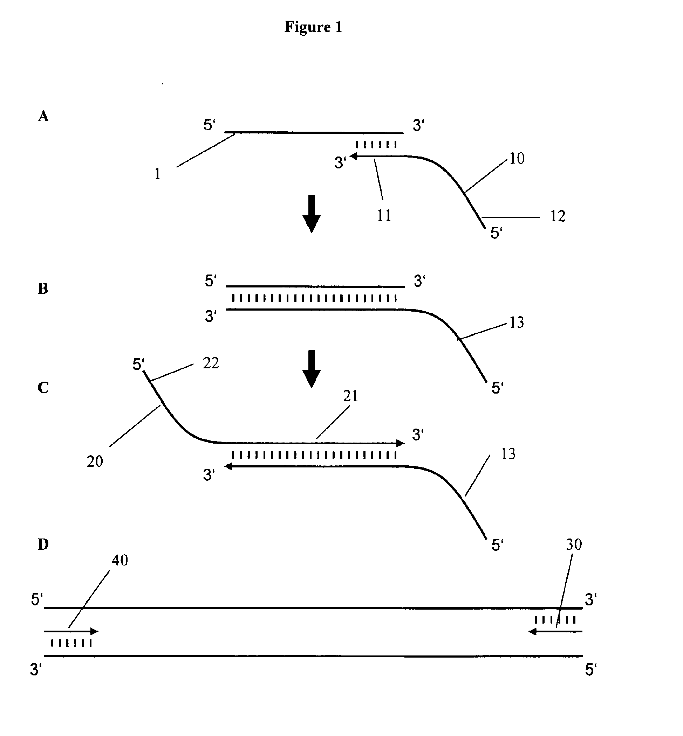

Figure 1 shows a schematic representation of the invention. Figure 1A depicts a first oligonucleotide molecule 10 that hybridizes at its 3' end with the 3' end of a small RNA molecule 1 (Fig. 1C). In the example shown, hybridization occurs over a sequence length of 6 nt. The sequence portion of the first oligonucleotide 10 that hybridizes with the small RNA molecule 1 is referred to as the first sequence portion 11 of the first oligonucleotide 10. The 5' end of the first oligonucleotide molecule 10, the second sequence portion 12, provides for a sequence that allows binding of a third oligonucleotide 30 to prime an enzymatic DNA amplification reaction, as will be explained below.

Binding of the first sequence portion 11 of the first oligonucleotide molecule 10 to the RNA template 1 allows for performing a reverse transcription reaction (also referred to as a primer extension reaction) in which the first oligonucleotide molecule 10 is extended at its 3' end, using the small RNA molecule 1 as a template. The result of this reverse transcription is shown in figure 1B, which shows that the first oligonucleotide molecule 10 has been extended to become a cDNA molecule 13.

The cDNA molecule 13 can then be used as a template for an enzymatic DNA amplification reaction. This reaction is performed in the presence of three oligonucleotides as primers. A second oligonucleotide molecule 20 comprises a first sequence portion 21 for hybridizing with the cDNA molecule and a second sequence portion 22 that provides for a sequence for binding of a fourth oligonucleotide 40 to prime the enzymatic DNA amplification reaction. After hybridization of the second oligonucleotide molecule 20 with the cDNA 13, a molecule is formed that has 5' overhangs on either side of the molecule. The 3' ends of each strand are then elongated to form a double stranded DNA molecule, which is depicted in figure 1D. A third oligonucleotide 30 as well as a fourth oligonucleotide 40 are used to amplify this double stranded DNA molecule. It is preferred that the third 30 and the fourth oligonucleotide 40 are constructed as universal primers, so that different small RNA molecules 1 can be analyzed using the same set of universal primers 30, 40.

In the enzymatic DNA amplification reaction step, the third and fourth oligonucleotide 30, 40 are preferably used in 10- to 50-fold excess, preferably in 20- to 30-fold excess, most preferably in 25-fold excess over the second oligonucleotide 20.

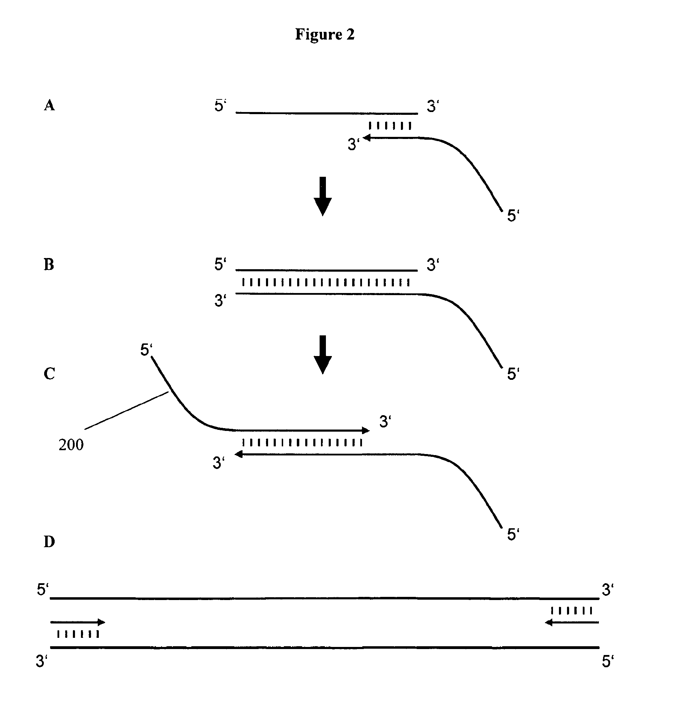

Figure 2 shows a method similar to the one depicted in figure 1. The only difference is that the second oligonucleotide molecule 200 used is shorter than the second oligonucleotide molecule 20 shown in figure 1.

Here, the second oligonucleotide molecule 200 reaches with its 3' end to the first nucleotide that was introduced in the primer extension step. In other words, the second oligonucleotide molecule 200 shown here is shortened with respect to the second oligonucleotide molecule 20 of figure 1 by the nucleotides that are contained within the first oligonucleotide and which hybridize to the RNA template.

It may, however, be advantageous that the second oligonucleotide molecule 200 is of such a length that it still hybridizes to the first sequence portion of the first oligonucleotide molecule 10. In such a case, it is preferred that the terminal 2 to 4, more preferably the terminal 3 nucleotides at the 3' end of the second oligonucleotide molecule 200 are reverse complementary to the terminal nucleotides at the 3' end of the first portion of the first oligonucleotide molecule 10.

The method shown in figure 2 is preferred for short RNA templates, such as miRNA, because it enhances the dynamic range further by one logarithmic step as compared to the longer second oligonucleotide of figure 1 and enhances the sensitivity of detection.

Therefore, in general terms, to construct a first oligonucleotide molecule, a sequence should be chosen that comprises a universal primer site at the 5' end and a range of 4 to 10 nt, preferably 6 nt, that hybridizes with the RNA template, preferably the 3' end of the RNA template in a sequence specific manner. The second oligonucleotide should comprise a universal primer site at the 5' end and a sequence that hybridizes with (preferably is reverse complementary to) the cDNA molecule generated in the reverse transcription reaction, which may exclude the sequence that is reverse complementary to the first to sixth nucleotide at the 3' end of the first oligonucleotide that was binding specifically to the RNA template (at least part of the first sequence portion of the first oligonucleotide).

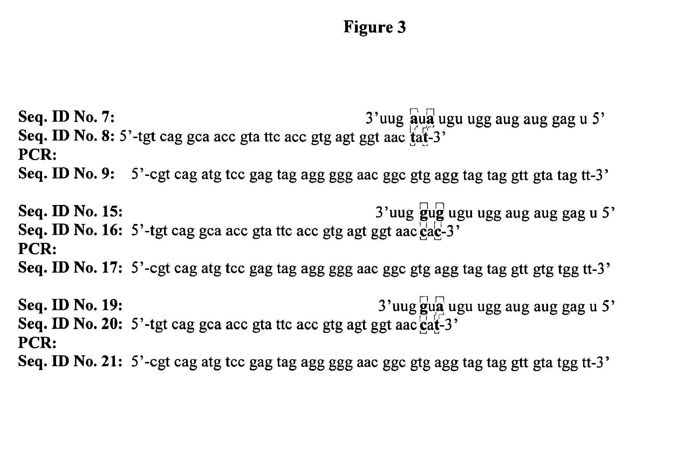

Figure 3 shows the analyzed members of the let-7 family with reference to example III. Bases with background indicate the differences between the miRNAs of the family.

### Examples

### Example I: miR-145

### Reverse transcription of miR-145 and quantitative PCR

The DNA- and RNA-oligonucleotides used are shown in table 1 and were synthesized by Metabion AG (Martinsried, Germany).

A first oligonucleotide (SEQ ID NO 2) comprises a first sequence portion (bold in table 1) at the 3' end and a second sequence portion (underlined in table 1) at the 5' end. The first sequence portion is six nucleotides (nt) in length and can hybridize with the 3' end of miR-145 (SEQ ID NO 1). Therefore, a sequence specific reverse transcription reaction (also referred to as primer extension reaction) can be performed. In this reaction, a cDNA molecule is synthesized that comprises a reverse complementary sequence to the template miR-145 RNA molecule in addition to a 5' overhang.

A miRNA-specific DNA-oligonucleotide (SEQ ID NO 2) with a 5'-overhang and the RevertAid™ M-MuLV Reverse Transcriptase (Fermentas GmbH, St. Leon-Roth, Germany) were used to transcribe miRNA into cDNA. The reaction was performed with 50 ng total RNA, 25 nmol/l of the first oligonucleotide (SEQ ID NO 2), 1 mmol/l dNTPs, 1x RT-Buffer, and 100 units RevertAid™ M-MuLV Reverse Transcriptase in a volume of 10 µl. The reaction was incubated at 42 °C for 60 min followed by an inactivation step at 70 °C for 10 min. 50 ng of E. coli total RNA spiked with different amounts of synthetic miRNA (100 fmol-0.1 amol) was applied for validation of new assays and for standard preparation. 50 ng of E. coli total RNA without synthetic miRNA represented the background.

Synthesized cDNA was measured using the Rotor-Gene 3000 Real-time Detection System (Corbett Life Science, Sydney, Australia). For this purpose, triplicate measurements of 3 µl cDNA were made in 10 µl final reaction volume. SYBR Green quantitative PCR (qPCR) was performed using the SensiMix DNA Kit (Quantace Ltd., Berlin, Germany), 4 nmol/l of a second oligonucleotide (SEQ ID NO 3), 100 nmol/l of a third oligonucleotide (SEQ ID NO 5, universal primer), and 100 nmol/l of a fourth oligonucleotide (SEQ ID NO 6, universal primer).

**Table 1: Oligonucleotides used in the examples (first sequence portions of first and second oligonucleotides are written in bold; second sequence portions of first and second oligonucleotides are underlined)**

| SEQ ID-NO: | Sequence (in 5' to 3' direction) | Name |
|---|---|---|
| 1 | guc cag uuu ucc cag gaa ucc cuu | miR-145 |
| 2 | tgt cag gca acc gta ttc acc gtg agt ggt **aag gga** | RT6-miR-145 |
| 3 | cgt cag atg tcc gag tag agg ggg aac ggc ggt cca gtt ttc cca gga **atc cct t** | miR-145-rev |
| 4 | cgt cag atg tcc gag tag agg ggg aac ggc ggt cca gtt ttc cca gga a | short- miR-145-rev |
| 5 | tgt cag gca acc gta ttc acc | MP-fw |
| 6 | cgt cag atg tcc gag tag agg | MP-rev |
| 7 | uga ggu agu agg uug u**au agu u** | let-7a |
| 8 | tgt cag gca acc gta ttc acc gtg agt ggt **aac tat** | RT6-let7a |
| 9 | cgt cag atg tcc gag tag agg ggg aac ggc gtg agg tag tag gtt gt**a tag tt** | let7a-rev |
| 10 | cgt cag atg tcc gag tag agg ggg aac ggc gtg agg tag tag gtt gta ta | short-let7a-rev |
| 11 | uag cuu auc aga cug aug uug a | miR-21 |
| 12 | tgt cag gca acc gta ttc acc gtg agt ggt **tca aca** | RT6- miR-21 |
| 13 | cgt cag atg tcc gag tag agg ggg aac ggc gta gct tat cag act ga**t gtt ga** | miR-21-rev |
| 14 | cgt cag atg tcc gag tag agg ggg aac ggc gta gct tat cag act ga | short-miR-21-rev |
| 15 | uga ggu agu agg uug ugu ggu u | let-7b |
| 16 | tgt cag gca acc gta ttc acc gtg agt ggt **aac cac** | RT6-let-7b |
| 17 | cgt cag atg tcc gag tag agg ggg aac ggc gtg agg tag tag gtt gtg tgg tt | let-7b-rev |
| 18 | cgt cag atg tcc gag tag agg ggg aac ggc gtg agg tag tag gtt gtg tg | short-let7b-rev |
| 19 | uga ggu agu agg uug uau ggu u | let-7c |
| 20 | tgt cag gca acc gta ttc acc gtg agt ggt **aac cat** | RT6-let-7c |
| 21 | cgt cag atg tcc gag tag agg ggg aac ggc gtg agg tag tag gtt gta tgg tt | let-7c-rev |
| 22 | cgt cag atg tcc gag tag agg ggg aac ggc gtg agg tag tag gtt gta tg | short-let-7c-rev |
| 23 | uag cag cac gua aau auu ggc g | miR-16 |
| 24 | tgt cag gca acc gta ttc acc gtg agt ggt **cgc caa** | RT6-miR-16 |
| 25 | cgt cag atg tcc gag tag agg ggg aac ggc gta gca gca cgt aaa tat tgg cg | miR-16-rev |
| 26 | cgt cag atg tcc gag tag agg ggg aac ggc gta gca gca cgt aaa ta | short-miR-16-rev |
| 27 | auc aca uug cca ggg auu acc | miR-23b |
| 28 | tgt cag gca acc gta ttc acc gtg agt get **ggt aat** | RT6-miR-23b |
| 29 | cgt cag atg tcc gag tag agg ggg aac ggc g atc aca ttg cca ggg att acc ag | miR-23b-rev |
| 30 | cgt cag atg tcc gag tag agg ggg aac ggc g atc aca ttg cca ggg | short-miR-23b-rev |
| 31 | uuc aca gug gcu aag uuc cgc | miR-27a |
| 32 | tgt cag gca acc gta **ttc** acc gtg agt ggt **gcg gaa** | RT6-miR-27a |
| 33 | cgt cag atg tcc gag tag agg ggg aac ggc gtt cac agt ggc taa gtt ccg c | miR-27a-rev |
| 34 | cgt cag atg tcc gag tag agg ggg aac ggc gtt cac agt ggc taa g | short-miR-27a-rev |
| 35 | uaa cac ugu cug gua aag aug g | miR-141 |
| 36 | tgt cag gca acc gta ttc acc gtg agt ggt **cca tct** | RT6-miR-141 |
| 37 | cgt cag atg tcc gag tag agg ggg aac ggc gta aca ctg tct ggt aaa gat gg | miR-141-rev |
| 38 | cgt cag atg tcc gag tag agg ggg aac ggc gta aca ctg tct ggt aa | short-miR-141-rev |
| 39 | uga gau gaa gca cug uag cuc a | miR-143 |
| 40 | tgt cag gca acc gta ttc acc gtg agt ggt **tga gct** | RT6-miR-143 |
| 41 | cgt cag atg tcc gag tag agg ggg aac ggc gtg aga tga agc act gta gct ca | miR-143-rev |
| 42 | cgt cag atg tcc gag tag agg ggg aac ggc gtg aga tga agc act gt | short-miR-143-rev |
| 43 | caa cgg aau ccc aaa agc agc u | miR-191 |
| 44 | tgt cag gca acc gta ttc acc gtg agt ggt **agc tgc** | RT6-miR-191 |
| 45 | cgt cag atg tcc gag tag agg ggg aac ggc gca acg gaa tcc caa aag cag ct | miR-191-rev |
| 46 | cgt cag atg tcc gag tag agg ggg aac ggc gca acg gaa tcc caa aa | short-miR-191-rev |
| 47 | uaa uac ugc cgg gua aug aug g | miR-200c |
| 48 | tgt cag gca acc gta ttc acc gtg agt ggt **cca tca** | RT6-miR-200c |
| 49 | cgt cag atg tcc gag tag agg ggg aac ggc gta ata ctg ccg ggt aat gat gg | miR-200c-rev |
| 50 | cgt cag atg tcc gag tag agg ggg aac ggc gta ata ctg ccg ggt aa | short-miR-200c-rev |

The third oligonucleotide (SEQ ID NO 5, universal primer), and a fourth oligonucleotide (SEQ ID NO 6, universal primer) bind to a sequence that is reverse complementary to the second sequence portion of the first and second oligonucleotide, respectively (underlined in table 1), which are present in the double stranded DNA molecule that results from extending the 3' ends after the first oligonucleotide molecule hybridizes with the second oligonucleotide molecule.

Unless otherwise indicated, amplification was carried out by running a first step at 95 °C for 10 min, followed by 40 cycles with 15 s at 95 °C and 30 s at 60 °C.

Melting points of the products obtained were determined. Measurements were performed by acquiring the fluorescent SYBR green signal of standards (synthetic miRNAs in the presence of 50 ng *E. coli* total RNA representing a complex background), control measurements (E. coli RNA, and H₂O) and ten porcine RNA samples, all in triplicate. Data is presented of the dynamic range of the assay of miRNA quantification. The standard concentration ranged from 100 fmol to 1 amol synthetic miR-145 (10⁻¹ dilutions) per reverse transcription reaction.

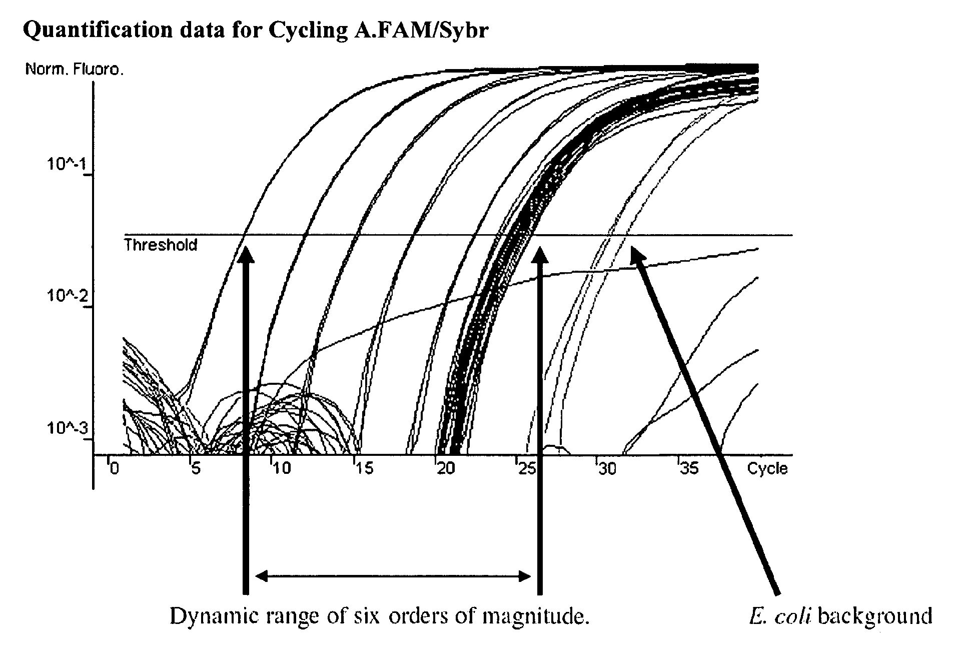

The shorter second oligonucleotide molecule (SEQ ID NO 4) is preferred over the second oligonucleotide molecule (SEQ ID NO 3), because it enhances the dynamic range of the present method, as can be seen from the following data obtained with said shorter second oligonucleotide molecule (SEQ ID NO 4). Dynamic ranges are indicated using an arrow. The standard concentration ranged from 100 fmol to 0.01 amol synthetic miR-145 (10⁻¹ dilutions) per reverse transcription reaction.

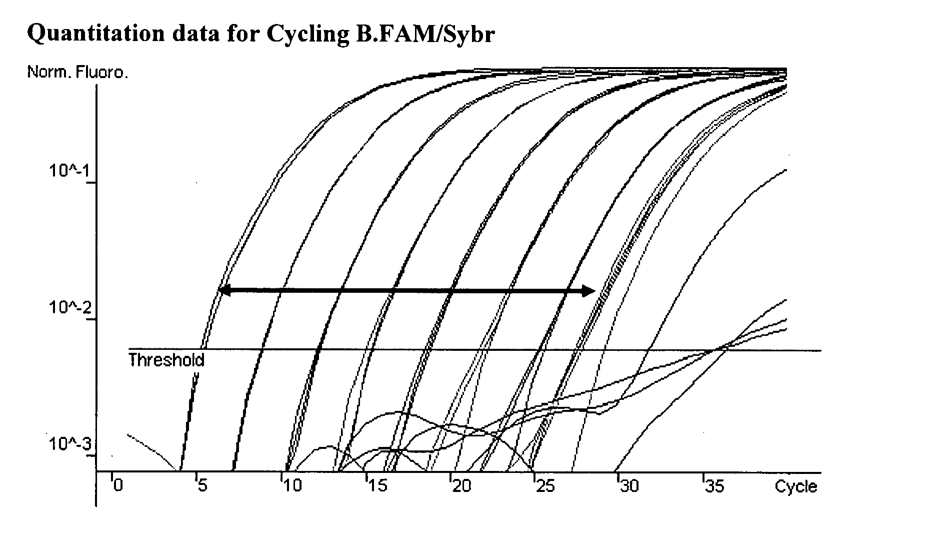
Quantitation data for Cycling B.FAM/Sybr
Norm. Fluoro.
10^-1
10^-2
Threshold
10^-3
0 | 5 | 10 | 15 | 20 | 25 | 30 | 35 | Cycle

### Example II: let-7a

Similar experiments were performed on let-7a RNA (SEQ ID NO 7) using a first oligonucleotide (SEQ ID NO 8), a second oligonucleotide (SEQ ID NO 9), and the pair of universal primers as described above (SEQ ID NOs 5 and 6). Instead of a second oligonucleotide molecule (SEQ ID NO 9), a shorter version (SEQ ID NO 10) can be used to increase the dynamic range of the present method, as indicated above.

### Example III: Discrimination between members of the let-7 family

The let-7 family consists of members that vary only in few numbers of nucleotides, as depicted in Figure 3. The ability to distinguish between different let-7 molecules by means of the present method was shown in cross-reaction experiments.

Representative studies were performed using the assays for let-7a (SEQ ID NO 7), let-7b (SEQ ID NO 15) and let-7c (SEQ ID NO 19). All experiments were done according to described procedures of reverse transcription of miRNA and quantitative PCR. Each assay was carried out with the specific synthetic miRNA and the other two members of the family. The cross-reaction of each assay with other analysed members of the family is indicated as percentage of the reaction with assay-specific miRNAs (Table 2).

### Reverse transcription of miRNA and quantitative PCR

The DNA- as well as RNA-oligonucleotides were synthesised by Metabion AG (Martinsried, Germany). The miRNA-specific DNA-oligonucleotide (first oligonucleotide molecule) with 5'-overhang and the RevertAid™ M-MuLV Reverse Transcriptase (Fermentas GmbH, St. Leon-Roth, Germany) were used to transcribe miRNA into cDNA. The reaction was performed with 50 ng total RNA, 25 nmol/l second oligonucleotide molecule, 1 mmol/l dNTPs, 1x RT-Buffer, and 100 units RevertAid™ M-MuLV Reverse Transcriptase in 10 µl.

A mixture of RNA and a first oligonucleotide molecule (SEQ ID NO 8, 16, 20, respectively) was first prepared in a 4 µl volume. The mixture was incubated at 70 °C for 5 min and chilled on ice. Then, the volume was brought to 10 µl by adding the buffer, dNTPs, Reverse Transcriptase and water. The reaction was incubated at 37 °C for 5 min followed by 42 °C for 60 min. The enzyme was inactivated by heating at 70 °C for 10 min. 50 ng of E. coli total RNA spiked with different amounts of synthetic miRNA (100 fmol-0.1 amol) was applied for validation of new assays and for standard preparation. 50 ng of E. coli total RNA without synthetic miRNA represented the background.

The synthesised cDNA was measured using the Rotor-Gene 3000 Real-time Detection System (Corbett Life Science, Sydney, Australia). For this purpose, triplicate measurements of 2-3 µl cDNA were made in 10 µl final reaction volume. SYBR Green quantitative PCR (qPCR) was performed using the SensiMix DNA Kit (Quantace Ltd., Berlin, Germany), 4 nmol/l of the second oligonucleotide molecule (SEQ ID NO 9, 17, 21, respectively), 100 nmol/l of the first universal primer (SEQ ID NO 5), and 100 nmol/l of the second universal primer (SEQ ID NO 5). Unless otherwise indicated, amplification was carried out by running a first step at 95 °C for 10 min followed by 40 cycles with 15 s at 95 °C and 30 s at 60 °C to 63 °C. Alternatively, a 3-step real-time PCR can be performed consisting of a first step at 95 °C for 10 min followed by 40 cycles with 15 s at 95 °C, 10-30 s at the particular annealing temperature, and 15 sec at 72 °C.

**Table 2: Values represent percentage of cross-reaction of each specific assay with other members of the let-7 family.**

| 30000 amol/qPCR | | | | | 30 amol/qPCR | | | |
|---|---|---|---|---|---|---|---|---|
| | synth. let-7a | synth. let-7b | symth. let-7c | | | synth. let-7a | synth. let-7b | synth. let-7c |
| let-7a Assay | 100 | 0.001 | 0.638 | | let-7a Assay | 100 | 0.03 | 0.389 |
| let-7b Assay | 0.183 | 100 | 0.045 | | let-7b Assay | 0.854 | 100 | - |
| let-7c Assay | 0.006 | 0.073 | 100 | | let-7c Assay | 0.006 | 0.05 | 100 |
| | | | | | | | | |

| 3000 amol/qPCR | | | | | 3 amol/qPCR | | | |
|---|---|---|---|---|---|---|---|---|
| | synth. let-7a | synth. let-7b | synth, let-7c | | | synth. let-7a | synth. let-7b | synth. let-7c |
| let-7a Assay | 100 | 0.002 | 0.4 | | let-7a Assay | 100 | 0.039 | - |
| let-7b Assay | 0.249 | 100 | 0.033 | | let-7b Assay | - | 100 | - |
| let-7c Assay | 0.005 | 0.046 | 100 | | let-7c Assay | 0.065 | | 100 |
| | | | | | | | | |

| 300 amol/qPCR | | | | | 0.3 amol/qPCR | | | |
|---|---|---|---|---|---|---|---|---|
| | synth. let-7a | synth. let-7b | synth. let-7c | | | synth. let-7a | synth. let-7b | synth. let-7c |
| let-7a Assay | 100 | 0.019 | 0.362 | | let-7a Assay | 100 | 5.371 | - |
| let-7b Assay | 0.318 | 100 | (0.084) | | let-7b Assay | - | 100 | - |
| let-7c Assay | 0.006 | 0.086 | 100 | | let-7c Assay | 0.712 | 1.776 | 100 |

### Example IV: miR-21

Similar experiments as described in example I were performed on miR-21 RNA (SEQ ID NO 11) using a first oligonucleotide (SEQ ID NO 12), a second oligonucleotide (SEQ ID NO 13), and the pair of universal primers as described above (SEQ ID NOs 5 and 6). Instead of a second oligonucleotide molecule (SEQ ID NO 13), a shorter version (SEQ ID NO 14) can be used to increase the dynamic range of the present method.

## Claims

1. A method for the quantitative analysis of RNA molecules, particularly small RNA molecules in a sample, comprising the following steps:
a) performing a reverse transcription reaction with a first oligonucleotide molecule (10) to yield DNA molecules that are reverse complementary in sequence to the RNA molecules (cDNA molecules) (13), wherein the first oligonucleotide molecule (10) comprises
- a first sequence portion (11) that hybridizes with the RNA molecules,
- a second sequence portion (12) that provides for a sequence for binding of a third oligonucleotide (30) to prime an enzymatic DNA amplification reaction,
b) performing an enzymatic DNA amplification reaction to amplify the cDNA molecules, comprising
- a second oligonucleotide molecule (20; 200), comprising a first sequence portion (21) for hybridizing with the cDNA molecules, and a second sequence portion (22) that provides for a sequence for binding of a fourth oligonucleotide (40) to prime the enzymatic DNA amplification reaction,
- a third oligonucleotide molecule (30) for priming an enzymatic DNA amplification reaction that hybridizes with a strand that is reverse complementary to the second sequence portion (12) of the first oligonucleotide (10), and
- a fourth oligonucleotide molecule (40) for priming an enzymatic DNA amplification reaction that hybridizes with a strand that is reverse complementary to the second sequence portion (22) of the second oligonucleotide (20; 200), and
c) performing a quantitative analysis of the amplified DNA molecules.

2. The method according to claim 1, wherein the second sequence portion (12) of the first oligonucleotide molecule (10) comprises a first universal primer binding site to prime an enzymatic amplification reaction.

3. The method according to claim 1 or 2, wherein the second sequence portion (22) of the second oligonucleotide molecule (20; 200) comprises a first universal primer binding site to prime an enzymatic amplification reaction.

4. The method according to any of claims 1 to 3, wherein the third oligonucleotide molecule (30) and/or the fourth oligonucleotide molecule (40) is a universal primer.

5. The method according to any of claims 1 to 4, wherein the first sequence portion (11) of the first oligonucleotide molecule (10) and the second sequence portion (12) of the first oligonucleotide molecule (10) are separate from each other.

6. The method according to any of claims 1 to 5, wherein at least the second sequence portion (12) of the first oligonucleotide molecule (10) forms a 5' overhang when the first oligonucleotide molecule is hybridized with an RNA molecule.

7. The method according to any of claims 1 to 6, wherein the first sequence portion (21) of the second oligonucleotide molecule (20; 200) and the second sequence portion (22) of the second oligonucleotide molecule (20; 200) are separate from each other.

8. The method according to any of claims 1 to 7, wherein the second sequence portion (22) of the second oligonucleotide molecule (20; 200) forms a 5' overhang when the second oligonucleotide molecule (20; 200) is hybridized with the cDNA molecule (13).

9. The method according to any of claims 1 to 8, wherein the enzymatic DNA amplification reaction is performed in the presence of a DNA-binding fluorescent dye or a probe that generates a real-time signal, the detection of which is used to quantitate the amount of RNA molecules in the sample.

10. The method according to any of claims 1 to 9, wherein the enzymatic DNA amplification reaction is a polymerase chain reaction (PCR).

11. The method according to any of claims 1 to 10, wherein the first sequence portion (11) of the first oligonucleotide molecule (10) is sequence specific for the RNA molecule to be analyzed.

12. The method according to any of claims 1 to 11, wherein more than one first oligonucleotide molecules (10) are used, which differ in their first sequence portion, to analyze more than one different RNA molecules simultaneously.

13. The method according to any of claims 1 to 12, wherein the RNA molecule is a microRNA (miRNA).

14. The method according to any of claims 1 to 13, wherein the quantitative analysis is performed by staining the amplified DNA molecules with an appropriate dye (e.g. SYBER green) and measuring the signals from the amplified DNA molecules.

15. Use of the method according to any of claims 1 to 14, for the diagnosis and/or prognosis of at least one disease, such as cancer.

16. A kit for the quantitative detection of RNA molecules, particularly small RNA molecules in a sample, comprising
- a first oligonucleotide molecule (10) comprising
- a first sequence portion (11) that hybridizes with the RNA molecules,
- a second sequence portion (12) that provides a sequence for binding of a third oligonucleotide (30) to prime an enzymatic DNA amplification reaction,
- a first enzyme catalyzing the reverse transcription of the RNA molecules into cDNA molecules (13)
- a second oligonucleotide molecule (20; 200), comprising
- a first sequence portion (21) for hybridizing with the cDNA molecules (13), and
- a second sequence portion (22) that provides a sequence for binding of a fourth oligonucleotide (40) to prime the enzymatic DNA amplification reaction,
- a second enzyme catalyzing an amplification reaction, and
- a third oligonucleotide molecule (30) for priming an enzymatic DNA amplification reaction,
- a fourth oligonucleotide molecule (40) for priming an enzymatic DNA amplification reaction.

17. The kit according to claim 16, wherein the first enzyme is a reverse transcriptase from *moloney murine leukemia virus.*

18. The kit according to claim 16 or 17, wherein the second enzyme is a DNA polymerase from *Thermus aquaticus.*

19. The kit according to any of claims 16 or 18, wherein at least the second sequence portion (12) of the first oligonucleotide molecule (10) forms a 5' overhang when the first oligonucleotide molecule (10) is hybridized with an RNA molecule.

20. The kit according to any of claims 16 or 19, wherein the second sequence portion (12) of the second oligonucleotide molecule (20; 200) forms a 5' overhang when the second oligonucleotide molecule (20; 200) is hybridized with the cDNA molecule (13) formed in a reverse transcription reaction using the first oligonucleotide (10).

21. An oligonucleotide for the quantitative analysis of RNA molecules, particularly small RNA molecules in a sample, selected from the group consisting of SEQ ID NOs 2 to 6, 8 to 10, 12 to 14, 16 to 18, 20 to 22, 24 to 26, 28 to 30, 32 to 34, 36 to 38, 40 to 42, 44 to 46, and 48 to 50.
